# EUROPEAN PATENT SPECIFICATION

(11) **EP 3 795 211 B1**
(45) Date of publication and mention of the grant of the patent: **09.11.2022**
(21) Application number: 21152956.5
(22) Date of filing: 22.01.2021
(51) Int. Cl.: A61N 1/30

(54) **SKIN LOTION INTRODUCING DEVICE**
VORRICHTUNG ZUM EINBRINGEN EINER HAUTLOTION
DISPOSITIF D'INTRODUCTION DE LOTION POUR LA PEAU

(30) Priority: 10.11.2020 CN 202011248885
(43) Date of publication of application: 24.03.2021
(73) Proprietor: Excelsior Medical Group, Kempston Bedford, Bedforshire MK42 7PW (GB)
(72) Inventor: Panesar, Bupinder, Bedford, Bedfordshire MK42 7PW (GB); Price, Richard, Bedford, Bedfordshire MK42 7PW (GB)
(74) Representative: Isern Patentes y Marcas S.L.

(56) References cited:
- EP-A1- 3 045 229
- CN-U- 206 963 994
- KR-A- 20140 076 742
- US-A1- 2005 191 252

## Description

### Technical Field

The present disclosure specifically relates to a skin lotion introducing device.

### Background Art

Along with the improvement of people's lives and the development of science and technology, nursing and beauty are required by people accordingly. However, due to aging and the environmental impacts, the metabolic rate of skin is slowed down, the activity of skin cells goes down, and issues such as dull and dry skin, large pores and the like are easy to occur. Cosmetic fluids are currently used for skin care. It is common to pour the cosmetic liquid directly onto the hand and manually apply it to the skin of human body, or to spray the cosmetic liquid onto the skin of the human body by placing it in a spraying device.

However, by applying the cosmetic liquid to the skin in the above-described manner, the absorption effect of the skin to the cosmetic liquid is poor, resulting in an unsatisfactory skin care effect of the cosmetic liquid. KR20140076742 discloses a magnetized water mist sprayer handpiece used in mist injection system of masseur machine assembly for health care, which has a magnet that is penetrated in a mist spray hole used for generating a magnetized water mist.

### Summary of the Disclosure

The present disclosure has been made in view of the mentioned state of the art, and the object of the present disclosure is to provide a skin lotion introducing device capable of effectively improving the absorption effect of the skin.

The invention is defined by claim 1. Preferred embodiments are defined in the dependent claims. Further aspects, examples and embodiments disclosed herein are for exemplary purpose only and do not form part of the invention.

According to the present disclosure, it is possible to provide a skin lotion introducing device capable of effectively improving the skin absorption effect.

### Brief Description of the Drawings

Embodiments of the present disclosure will now be explained in further detail, by way of example only, with reference to the accompanying drawings, in which:
Figure 1 is a schematic diagram illustrating an application of the introducing device according to an embodiment of the present disclosure.
Figure 2 is a schematic diagram illustrating a structure of the introducing device according to an embodiment of the present disclosure.
Figure 3 is a schematic diagram illustrating a structure of the stimulation head according to an embodiment of the present disclosure.
Figure 4 is a block diagram illustrating a structure of the introducing device according to an embodiment of the present disclosure.
Figure 5 is a block diagram illustrating a structure of the spraying device according to an embodiment of the present disclosure.
Figure 6 is a schematic diagram illustrating a structure of the base according to an embodiment of the present disclosure at different viewing angles.
Figure 7 is a block diagram illustrating a structure of the base according to an embodiment of the present disclosure.
Figure 8 is a schematic diagram illustrating a structure of the introducing device according to an embodiment of the present disclosure.
Figure 9 is a block diagram illustrating a structure of the introducing device according to another embodiment of the present disclosure.

### Detailed Description of the Disclosure

The present disclosure will now be described in further detail with reference to the accompanying drawings and specific embodiments. In the drawings, the same reference numerals are used for the same parts or parts having the same function, and a repeated description thereof will be omitted.

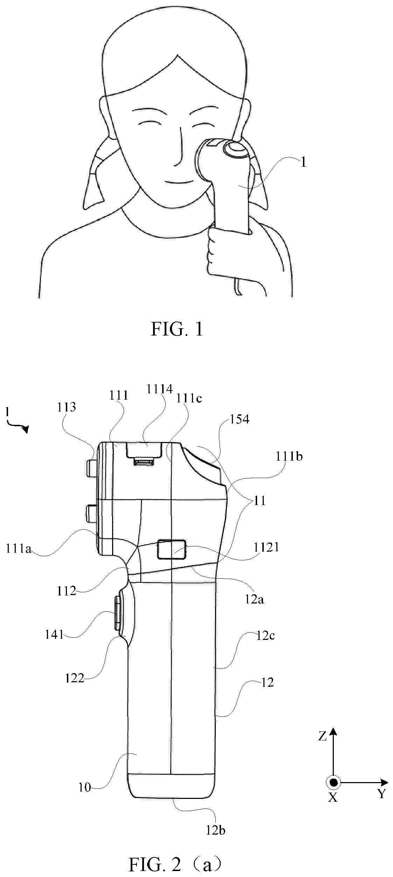

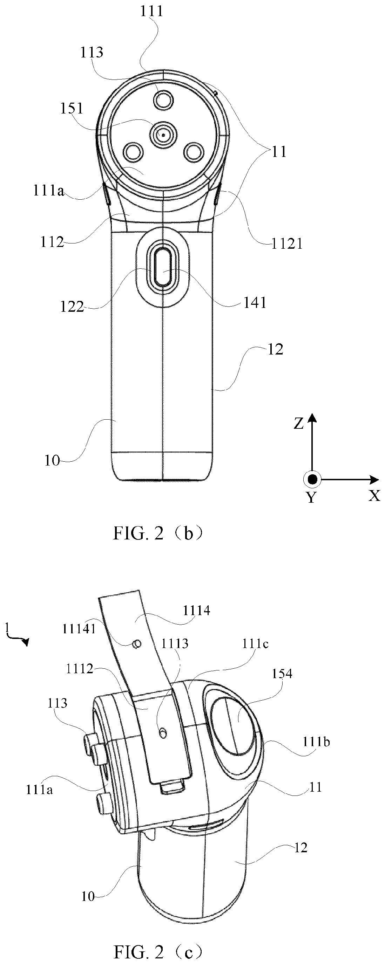

The present disclosure provides a skin lotion introducing device 1 (see Figures 1 and 2). Embodiments of the present disclosure relate to an introducing device 1 that can both spray a skin lotion onto a user's skin for skin absorption, and electrically stimulate the user's skin using an iontophoresis technology to promote the skin to absorb the skin lotion attached to the surface of the skin more effectively, etc. Wherein the skin lotion may be a nutrient solution for skin absorption, such as an essence for moisturizing the skin, whitening, etc.

The introducing device 1 according to the present disclosure is described in detail hereinafter with reference to the accompanying drawings.

Figure 1 is a schematic diagram illustrating an application of the introducing device 1 according to an embodiment of the present disclosure.

In some examplary embodiments, the introducing device 1 may be handheld for use by a user (see Figure 1). During use of the introducing device 1, the introducing device 1 may be configured in a spraying mode for spraying a skin lotion onto the surface of the skin of a user or an introducing mode for electrically stimulating the skin. Specifically, when the introducing device 1 is used, a user can hold the introducing device 1 in hand so that the introducing device 1 approaches the skin surface to be treated (e.g., face surface, etc.), and then the skin lotion can be uniformly sprayed on the surface of the skin (i.e., the spraying mode), or the skin can be electrically stimulated with the introducing device 1 to enhance an absorption effect of the skin (i.e., the introducing mode).

Figure 2 is a schematic diagram illustrating a structure of the introducing device 1 according to an embodiment of the present disclosure. Herein, Figure 2(a) is a perspective structural diagram of the introducing device 1. Figures 2(b) and 2(c) are perspective structural diagrams of the introducing device 1 of Figure 2(a) at two different viewing angles, respectively. In the embodiment shown in Figure 2, a detailed description can be made on the basis of an XYZ orthogonal coordinate system (see Figure 2 (a)). The direction X may correspond to horizontal left-right direction, the direction Y may correspond to horizontal front-rear direction, and the direction Z may correspond to vertical up-down direction. The direction Y and the direction Z are orthogonal to the direction X, respectively. Sometimes direction X is labeled left or right, direction Y is labeled front or rear, and direction Z is labeled up or down.

In embodiments to which this disclosure relates, the shape of the main body shell 10 may not be limited so much, wherein the main body shell 10 may be substantially formed in shape that facilitates handholding. Hereinafter related embodiments of the main body shell 10 will be described in detail with reference to the accompanying drawings.

In some examplary embodiments, the introducing device 1 may include a main body shell 10 (see Figures 1 and 2). In some examplary embodiments, the main body shell 10 may include a head part 11 and a holding part 12 serving as handle (see Figure 2). Herein, the holding part 12 may be connected to the head part 11. In this case, the introducing device 1 can be hand-held used by a user. In some examplary embodiments, the main body shell 10 may have a hollow structure. For example, head part 11 may have a first hollow structure, the holding part 12 may have a second hollow structure, and the first and second hollow structures may communicate with each other to form a hollow structure of main body shell 10. In some examplary embodiments, the main body shell 10 may be formed from a resin material such as PS757K ABS plastic through molding process.

In some examplary embodiments, the head part 11 may include a functional portion 111 and a connecting portion 112 connected to the functional portion 111 (see Figure 2).

In some examplary embodiments, the functional portion 111 may be substantially hollow (or "have an interior cavity") and collumnar. For example, as shown in Figure 2, the functional portion 111 may be substantially cylindrical. In some examplary embodiments, the connecting portion 112 may be formed at an outer periphery 111c of the functional portion 111. In some examplary embodiments, the connecting portion 112 may be provided to protrude downward from lower surface of the rear part of the outer periphery 111c of the function portion 111 (see Figure 2). For example, in some examplary embodiments, the connecting portion 112 may be substantially cylindrical with a through hole. In some examplary embodiments, the through hole of the connecting portion 112 may communicate with the inner cavity of the functional portion 111 to form the first hollow structure.

In some examplary embodiments, the connecting portion 112 may be integrally formed with the functional portion 111. In other embodiments, the connecting portion 112 and the functional portion 111 may be connected to each other by other means such as gluing.

In some examplary embodiments, the connecting portion 112 may be connected to the holding part 12. In some examplary embodiments, at the connection area of the connecting portion 112 and the holding part 12, the connecting portion 112 may have the same horizontal cross-section as the holding part 12.

In some examplary embodiments, the rear surface 111b of the functional portion 111 may be formed in a curved shape. In some examplary embodiments, the shape of the rear surface 111b may be substantially the same as the shape of the holding part 12 (described later).

In some examplary embodiments, the head part 11 may have a stimulation head 113 for stimulating the skin. In some examplary embodiments, the stimulation head 113 may be disposed on a front surface 111a of the function portion 111. In some examplary embodiments, the stimulation head 113 may protrude from the front surface 111a (see Figure 2). In some examplary embodiments, the stimulation head 113 may be made of metal or other conductive material. For example, the stimulation head 113 may be made of 304 stainless steel. In some examplary embodiments, the number of stimulation heads 113 may be one or more. In some examplary embodiments, the protrusion heights of a plurality of stimulation heads 113 with respect to the front surface 111a may be the same. Thereby, the stimulation head 113 can stimulate the skin more effectively.

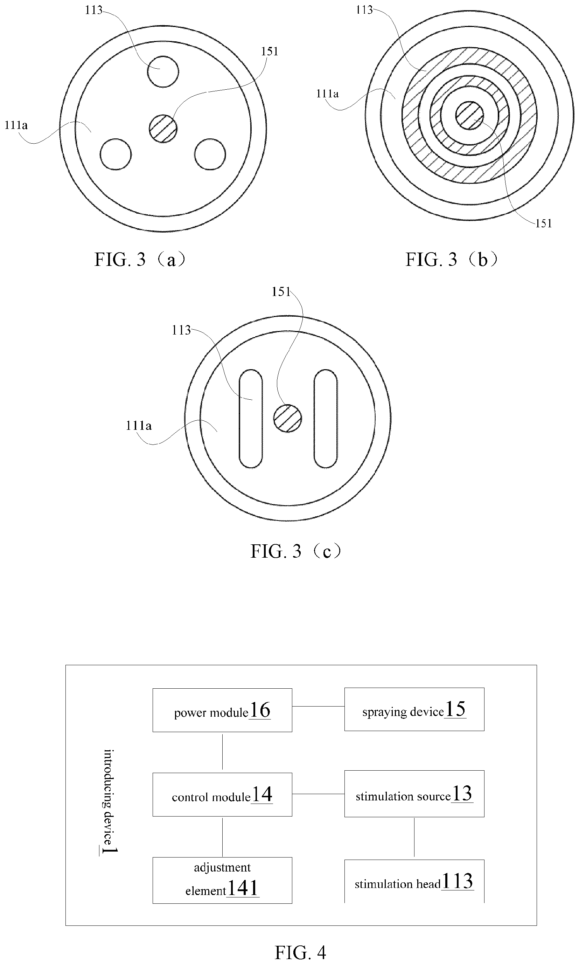

Figure 3 is a schematic diagram illustrating a structure of the stimulation head 113 according to an embodiment of the present disclosure.

In some examplary embodiments, the stimulation head 113 may be a plurality of point electrodes (see Figure 3(a)). In some examplary embodiments, the plurality of point electrodes may be uniformly distributed on the front surface 111a.

In some examplary embodiments, the stimulation head 113 may be a plurality of ring electrodes (see Figure 3(b)). In some examplary embodiments, the plurality of ring electrodes may be distributed on the front surface 111a concentrically.

In some examplary embodiments, the stimulation head 113 may be a plurality of strip electrodes (see Figure 3(c)). In some examplary embodiments, the plurality of strip electrodes may be distributed parallel to each other on the front surface 111a.

In some examplary embodiments, when the user holds the introducing device 1 in hand to electrically stimulate the skin to be treated, the stimulation head 113 may contact and electrically stimulate the surface of the skin. In some examplary embodiments, the stimulation head 113 may apply electrical stimulation of appropriate strength to the skin, and may form temporary, reversible hydrophilic channels (i.e., hydrophilic channels) in lipid bilayers such as cell membranes. Therefore, the permeability of skin cells and tissue membranes can be increased, and a good absorption effect of nutritional ingredients in the skin lotion can be achieved. In some examplary embodiments, the end of the stimulation head 113 near the surface of the skin may be formed in a curved shape. For example, if the stimulation head 113 is point electrodes equipped, the end of the stimulation head 113 may be formed in spherical shape.

In some examplary embodiments, the holding part 12 may be substantially hollow and collumnar. For example, the holding part 12 may be cylindrical or prismatic, etc. In some examplary embodiments, the cross-section of the holding part 12 in the horizontal direction may be a circle or an ellipse, etc. In some examplary embodiments, an upper end surface 12a of the holding part 12 may be no smaller than a lower end surface 12b thereof. In some examplary embodiments, the transition between the upper end face 12a and the lower end face 12b may be smooth. Thus, the holding part 12 can be formed to facilitate hand-holding.

In some examplary embodiments, the upper end face 12a may be closer to the functional portion 11 than the lower end face 12b. For example, the upper end surface 12a may be the connecting surface of the connecting portion 112 and the holding part 12.

In some examplary embodiments, the holding part 12 and the connecting portion 112 may be mechanically detachably connected to each other. For example, in some examplary embodiments, the holding part 12 and the connecting portion 112 may be provided with cooperating snap-fit structures. In this case, the user can easily adjust and examine the inside of the introducing device 1. In some examplary embodiments, the connection between the holding part 12 and the connecting portion 112 may be controlled by a plurality of docking keys 1121. For example, the user may press and adjust the docking keys 1121 to separate the connecting portion 112 from the holding part 12, or to assemble the connecting portion 112 with the holding part 12. In some examplary embodiments, if there are multiple docking keys 1121, a user may simultaneously operate multiple docking keys 1121 for disassembling or assembling. In some examplary embodiments, the docking keys 1121 may be disposed on the outer periphery, close to the holding part 12, of the head part 11. For example, the docking keys 1121 may be disposed on the connecting portion 112. Alternatively, the docking keys 1121 may be provided on the outer periphery 12c, close to the head part 11, of the holding part 12.

In other embodiments, the holding part 12 may be integrally formed with the connecting portion 112. Alternatively, the connecting portion 112 and the holding part 12 may be connected to each other by other means such as gluing.

Figure 4 is a block diagram illustrating a structure of the introducing device 1 according to an embodiment of the present disclosure.

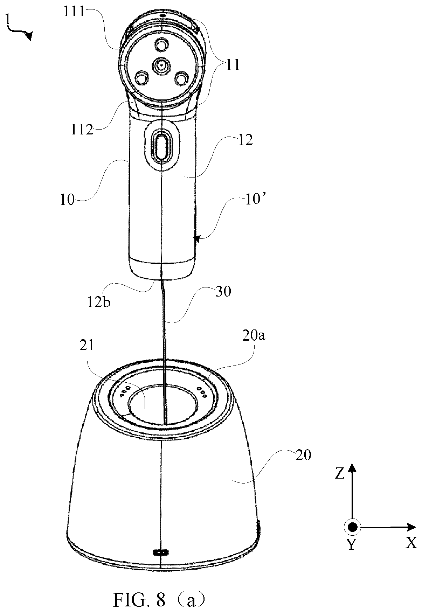

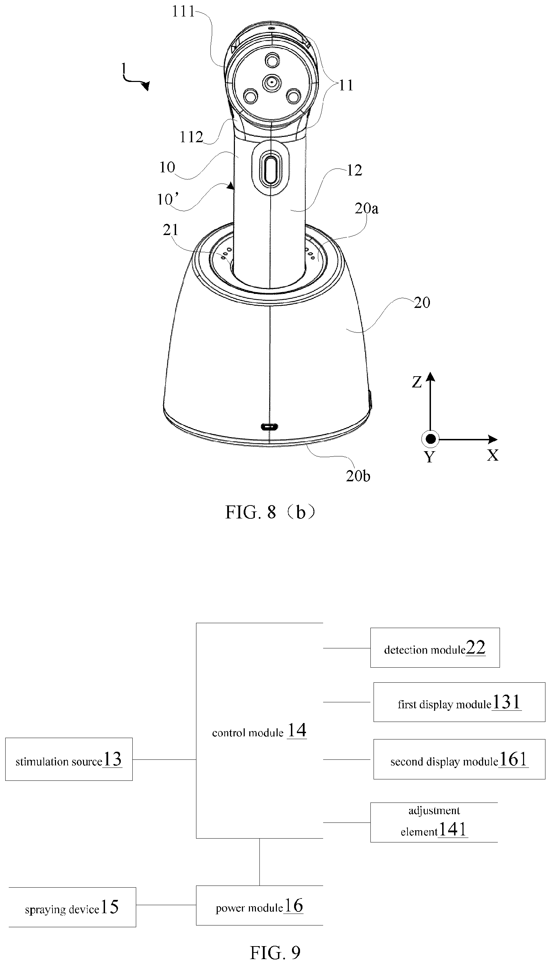

In some examplary embodiments, when the introducing device 1 is configured to be the introducing mode, the stimulation head 113 needs to apply electrical stimulation to the skin, for which purpose the introducing device 1 may also have a stimulation source 13 for providing electrical stimulation to the stimulation head 113 (see Figure 4). In some examplary embodiments, the stimulation source 13 may be connected to the stimulation head 113. In some examplary embodiments, the stimulation source 13 may be an electro-pulse generator. Thus, the stimulation source 13 can generate electric pulse and transmit to the stimulation head 113 so that the stimulation head 113 electrically stimulates the contacted skin. In some examplary embodiments, the stimulation source 13 may be disposed inside the introducing device 1. For example, in some examplary embodiments, the stimulation source 13 may be disposed within a hollow structure of the main body shell 10. In some examplary embodiments, the stimulation source 13 may also be disposed within the base 20 (described in more detail below). Thereby, the weight of the handheld device 10 ' (see Figure 8) can be effectively reduced.

In some examplary embodiments, the stimulation source 13 may be connected to a power module 16 (described in more detail below) to enable the power module 16 to supply power to the stimulation source 13.

In some examplary embodiments, the stimulation source 13 may generate electrical stimulation of different intensities. For example, in some examplary embodiments, the pulse frequency of the electric pulses generated by the stimulation source 13 may be 1.5 kHz-3 KHz. For example, the pulse frequency of the electric pulses generated by the stimulation source 13 may be 1.8 kHz, 2 kHz, 2.2 kHz, 2.4 kHz, or 2.5 kHz, etc.

In some examplary embodiments, the maximum pulse current of the electric pulses generated by the stimulation source 13 may be 0-10 mA. For example, the maximum pulse current of the electric pulses generated by stimulation source 13 may be 0.5 mA, 1 mA, 1.5 mA, 2 mA, 2.5 mA, 3 mA, 4 mA, 5 mA, 6 mA, 7 mA, 8 mA, 9 mA, or 10 mA, etc.

In some examplary embodiments, the pulse amplitude of the electric pulses generated by the stimulation source 13 may be 20-80 V For example, the pulse amplitude of the electric pulses generated by the stimulation source 13 may be 36 V, 38 V, 40 V, 42 V, 44 V, 50 V, 55 V, 60 V, 65 V, 70 V, 75 V, or 80 V, etc.

In some examplary embodiments, the introducing device 1 may also have a control module 14 (see Figure 4) connected to the stimulation source 13. In some examplary embodiments, the intensity of the electric stimulation generated by the stimulation source 13 may be controlled by the control module 14, i.e., the control module 14 may control an output state of the stimulation source 13. In particular, the control module 14, in operation, may generate and transmit control signals to the stimulation source 13, which may receive the control signals and adjust the intensity of the emitted electric stimulation in accordance with the control signals. In some examplary embodiments, the control module 14 may be arranged inside the introducing device 1. For example, the control module 14 may be disposed within the main body shell 10 (i.e., within the hollow structure). Alternatively, the control module 14 may be disposed within the base 20.

In some examplary embodiments, the control module 14 may be connected to a power module 16 (described in more detail below) to enable the power module 16 to supply power. In some examplary embodiments, the control module 14 may also be used to detect the power module 16 to obtain working state and the amount of electricity information, etc. (described in more detail below) of the power module 16. Therefore, the control module 14 can acquire the working state and the amount of electricity information of the power module 16, and the subsequent adjustment of the introducing device 1 can be facilitated.

In some examplary embodiments, the introducing device 1 may also have an adjustment element 141 (see Figures 2 and 4) connected to the control module 14. In some examplary embodiments, the adjustment element 141 may be a mechanical switch having mechanical contacts. Alternatively, the adjustment element 141 may be an electronic switch that detects finger contact and switches electronically. In some examplary embodiments, the adjustment element 141 may be disposed on an outer wall of the introducing device 1. For example, in some examplary embodiments, referring to Figure 2, the holding part 12 may be provided with a protruding stage 122 close to the outer periphery 12c of head part 11. The protruding stage 122 is provided with a window for mounting the adjustment element 141. The adjustment element 141 may be a key switch. The adjustment element 141 may be mounted at the window by means of horizontal pressing (direction Y).

In some examplary embodiments, the control module 14 may be enabled to control and adjust the output state of the stimulation source 13 by adjusting the adjustment element 141. Specifically, by adjusting the adjustment element 141 with a predetermined adjustment mode, the control module 14 can be enabled to control and adjust the stimulation source 13 into a corresponding output state. For example, in some examplary embodiments, the stimulation source 13 may be in a state in which output is stopped (i.e., an off state) before the introducing device 1 is configured in the introducing mode. The user can configure the introducing device 1 in the introducing mode, i.e. the stimulation source 13 is in an outputable state, i.e. the on-state, by long pressing the adjustment element 141. In some examplary embodiments, multiple different levels may be set in the introducing mode. Different levels may correspond to different output states of the stimulation source 13. That is, respective levels of the introducing mode may be divided according to the intensity of the electrical stimulation generated by the stimulation source 13. In some examplary embodiments, when the intensity of the electrical stimulation generated by the stimulation source 13 is high (i.e., a high output state), the corresponding level of the introducing mode is a high level. When the intensity of the electrical stimulation generated by the stimulation source 13 is low, the corresponding level of the introducing mode is a low level (i.e., a low output state).

In some examplary embodiments, when the introducing device 1 is configured in the introducing mode by long pressing of the adjustment element 141, the stimulation source 13 may be in a low output state, i.e. corresponding to a low level. The output state of the stimulation source 13 may then be changed by adjusting the adjustment element 141. For example, the user may adjust the output state of the stimulation source 13 by pressing (e.g., short pressing) the adjustment element 141. In some examplary embodiments, after the use of the introducing mode of the introducing device 1 is completed, the user may place the stimulation source 13 in an off state by adjusting (e.g., long pressing) the adjustment element 141.

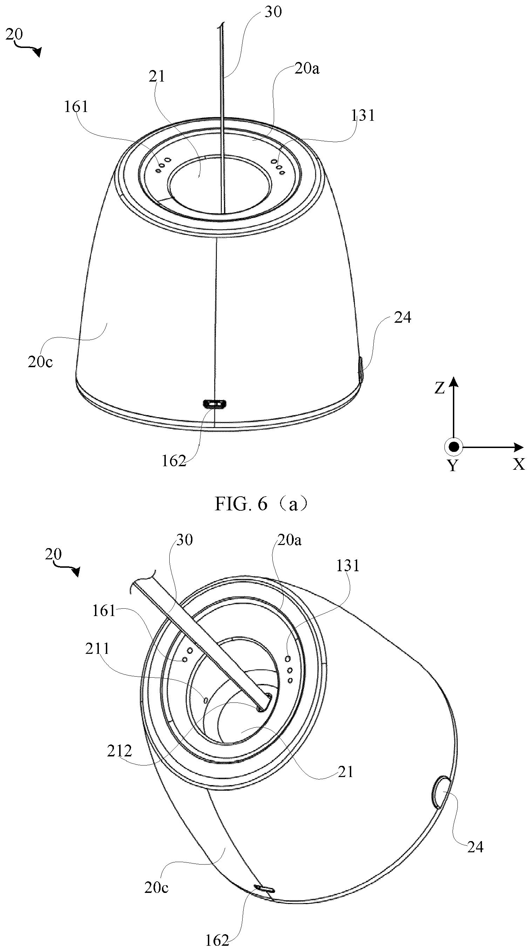

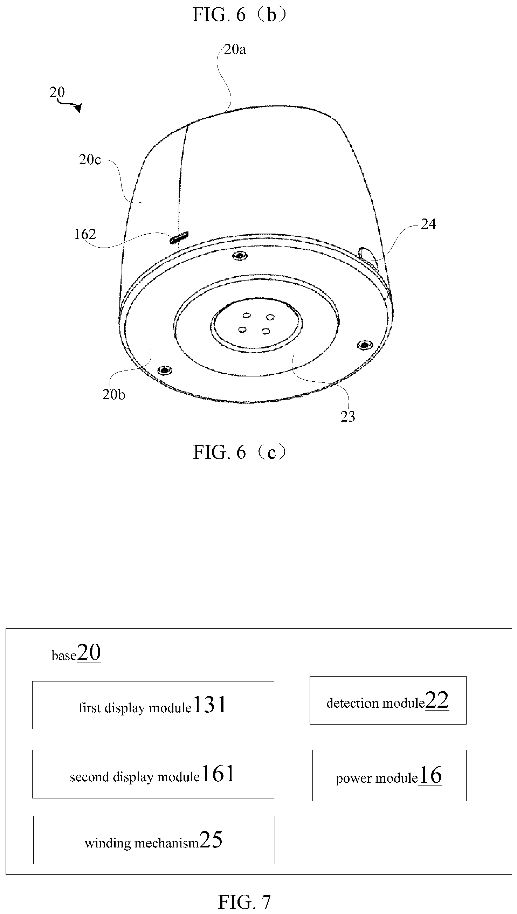

In some examplary embodiments, the introducing device 1 may also have a first display module 131 for reflecting the output state of the stimulation source 13. In some examplary embodiments, the first display module 131 may be connected to the control module 14 and/or the stimulation source 13 to obtain and display the output state of the stimulation source 13. In some examplary embodiments, the first display module 131 may include a plurality of first display lights (see Figure 6) reflecting the output state of the stimulation source 13 by the number of lighted lights in the first display lights. Specifically, the higher the corresponding level is, i.e., the higher the intensity of the electric stimulation generated by the stimulation source 13 is, the higher the number of lighted lights in the plurality of first display lights is. For example, the first display module 131 may include three second display lights that are all off if the stimulation source 13 is off. If the stimulation source 13 is in a low output state, one of the three second display lights is in a bright state. If the stimulation source 13 is in a medium output state, two of the three second display lights are in a bright state. If the stimulation source 13 is in a high output state, then all three second display lights are in a bright state. Wherein, the medium output state means that the intensity of the electrical stimulation generated by the stimulation source 13 is located between the low output state and the high output state.

In other embodiments, the first display module 131 may also be other display devices. For example, the first display module 131 may include a display panel on which the output state of the stimulation source 13 is directly displayed in digits or characters, etc.

In some examplary embodiments, the first display module 131 may be disposed on the main body shell 10 (not shown). Alternatively, the first display module 131 may be disposed on the base 20 (see Figure 6). For example, referring to Figure 6, the first display module 131 may be disposed on an end face 20a of the base 20.

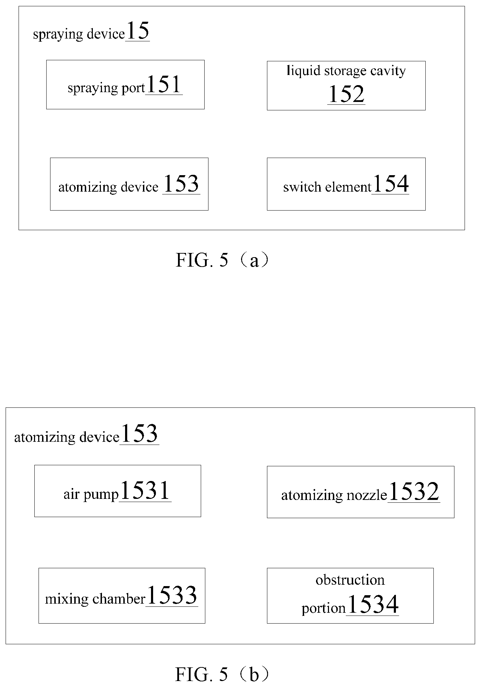

Figure 5 is a block diagram illustrating a structure of the spraying device 15 according to an embodiment of the present disclosure. Herein, Figure 5(a) is a block diagram illustrating an overall structure of the spraying device 15. Figure 5(b) is a block diagram illustrating a structure of the atomizing device 153 in the spraying device 15.

In some examplary embodiments, the introducing device 1 may also be configured in a spraying mode in which the user holds the introducing device 1 in hand to spray the skin lotion onto the skin to be treated, for which purpose the introducing device 1 may also have a spraying device 15 for spraying the skin lotion onto the surface of the skin (see Figures 4 and 5).

In some examplary embodiments, the spraying device 15 may have a spraying port 151. In some examplary embodiments, the skin lotion may be sprayed through the spraying port 151 toward the surface of the skin. Specifically, when the introducing device 1 is configured in a spraying mode, a user can hold the introducing device 1 in a hand and close the spraying port 151 to the surface of the skin to be treated, and spray the skin lotion toward the surface of the skin.

In some examplary embodiments, the spraying port 151 may be disposed on the front surface 111a of the functional portion 111. For example, the spraying port 151 may be disposed at a central position of the front surface 111a. In some examplary embodiments, the spraying port 151 and the stimulation head 113 may be located at different locations on the front surface 111a, respectively. In some examplary embodiments, the spraying port 151 may not protrude from the front surface 111a in the Y direction. In other embodiments, if the spraying port 151 protrudes from the front surface 111a, the height at which the spraying port 151 protrudes from the front surface 111a may be less than that of the stimulation head 113. In this case, when the stimulation head 113 electrically stimulates the skin, the influence of the spraying port 151 on the stimulation head 113 can be reduced.

In some examplary embodiments, the spraying device 15 also has a liquid storage cavity 152 for accomodating the skin lotion. In some examplary embodiments, the liquid storage cavity 152 may be in communication with the spraying port 151. In this case, when the introducing device 1 is configured in the spraying mode, the skin lotion may be sprayed from the liquid storage cavity 152 through the spraying port 151 to the surface of the skin.

In some examplary embodiments, the liquid storage cavity 152 may be disposed inside the introducing device 1. For example, in some examplary embodiments, the liquid storage cavity 152 may be disposed internally within the main body shell 10. In some examplary embodiments, the liquid storage cavity 152 may be detachably disposed inside the introducing device 1. In this case, when the head part 11 and the holding part 12 are detached, the liquid storage cavity 152 can be removed from the main body shell 10 for cleaning or skin lotion refilling or the like. In some examplary embodiments, the height of the liquid storage cavity 152 may match the height of the spraying port 151. For example, the height of the liquid storage cavity 152 may be higher than that of the spraying port 151. That is, the liquid storage cavity 152 is closer to the upper side than the spraying port 151.

In some examplary embodiments, the spraying device 15 may also have an atomizing device 153 (see Figures 5(a) and 5(b)). In some examplary embodiments, when the introducing device 1 is configured in the spraying mode, the atomizing device 153 may enable the skin lotion in the vicinity of the spraying port 151 to be sprayed from the spraying port 151 to the surface of the skin in an atomized form. Thus, the skin can absorb the skin lotion more easily.

In some examplary embodiments, the atomizing device 153 may be partially disposed within the introducing device 1. For example, in some examplary embodiments, the atomizing device 153 may be partially disposed within the main body shell 10. In some examplary embodiments, the atomizing device 153 may include an air pump 1531. In some examplary embodiments, the air pump 1531 may be disposed inside the main body shell 10. In some examplary embodiments, the atomizing device 153 may also include an atomizing nozzle 1532 disposed at the spraying port 151. In some examplary embodiments, the atomizing nozzle 1532 may protrude forward from the front surface 111a. That is, in the Y direction, the atomizing nozzle 1532 may not protrude from the front surface. In other embodiments, if the atomizing nozzle head protrudes from the front surface 111a, the height at which the atomizing nozzle protrudes from the front surface 111a may be less than that of the stimulation head 113. In this case, when the stimulation head 113 electrically stimulates the skin, the influence of the atomizing nozzle on the stimulation head 113 can be reduced. In this case, when the introducing device 1 is configured in the spraying mode, the air pump may pressurize the skin lotion to be sprayed through the atomizing nozzle 1532 located at the spraying port 151 so that it is sprayed to the surface of the skin in an atomized form.

In some examplary embodiments, the atomizing device 153 also includes a mixing chamber 1533 disposed below the liquid storage cavity 152. In some examplary embodiments, the liquid storage cavity 152 may be in communication with the mixing chamber 1533. In this case, the skin lotion in the liquid storage cavity 152 may enter the mixing chamber 1533. In some examplary embodiments, the communication of the liquid storage cavity 152 with the mixing chamber 1533 may also be provided with a restriction module (not shown) for restricting skin care fluid communication. In some examplary embodiments, when the introducing device 1 is configured in the spraying mode, the restriction module may be opened to allow skin care fluid in the liquid storage cavity to enter the mixing chamber 1533.

In some examplary embodiments, the air pump 1531 may be disposed below the mixing chamber 1533. In some examplary embodiments, the air pump 1531 and the mixing chamber 1533 may be provided with gas passages. In some examplary embodiments, when the introducing device 1 is configured in the spraying mode, the air pump 1531 may generate pressurized gas which enters the mixing chamber 1533 from the gas passage to pressurize the skin care fluid in the mixing chamber 1533 to be ejected from the spraying port 151. In some examplary embodiments, the introducing device 1 also has a restriction portion (not shown) for restricting the skin lotion within the mixing chamber 1533 from entering the gas passageway. In some examplary embodiments, the restriction portion may be a restriction membrane disposed at an outlet of the gas passage. The restriction membrane may allow only gas to pass through.

In some examplary embodiments, the atomizing device 153 also has an obstruction portion1534 disposed inside the main body shell 10 for shielding the spraying port 151. In some examplary embodiments, the obstruction portion 1534 may be a movable thimble disposed in the mixing chamber 1533. In some examplary embodiments, when the introducing device 1 is configured in the spraying mode (e.g., when the switch element 154 is pressed), the obstruction portion 1534 may open the spraying port 151 so that the skin lotion may be sprayed through the spraying port 151 to the surface of the skin. When the introducing device 1 is not configured in the spraying mode, the obstruction portion 1534 may shield the spraying port 151. In this case, it is possible to suppress the skin lotion from passing through the spraying port 151.

In some examplary embodiments, the atomizing device 153 may also include an atomizing nozzle 1532 disposed at the spraying port 151. In some examplary embodiments, if the atomizing nozzle protrudes from the front surface 111a, the height at which the atomizing nozzle protrudes from the front surface 111a may be less than that of the stimulation head 113. In this case, when the stimulation head 113 electrically stimulates the skin, the influence of the atomizing nozzle on the stimulation head 113 can be reduced.

In some examplary embodiments, the atomizing device 153 may be connected to the power module 16 (described in more detail below) to enable the power module 16 to supply power. In some examplary embodiments, the atomizing device 153 may also be connected to the control module 14 (described in more detail below). The control module 14 may adjust the working state of the atomizing device 153.

In some examplary embodiments, the spraying device 15 is also provided with a switch element 154 for controlling the spraying. In some examplary embodiments, the switch element 154 may be a mechanical switch having mechanical contacts. Alternatively, the switch element 154 may be an electronic switch that detects finger contact and switches electronically. In some examplary embodiments, the switch element 154 may be disposed on an outer wall of the introducing device 1. For example, in some examplary embodiments, with reference to Figure 2, a window for mounting the switch element 154 is provided above and at the rear of the functional portion 111. The switch element 154 may be a key switch. The switch element 154 may be mounted at the window in a vertically pressed (direction Z) manner.

In some examplary embodiments, the spraying device 15 may also be connected to and controlled to spray out by the control module 14. Specifically, the control module 14 may send an spraying control signal for controlling spraying to the spraying device 15, and the spraying device 15 may receive the spraying control signal and determine whether to spray or not, etc., on the basis of the signal. In some examplary embodiments, the switch element 154 may be the same element as the adjustment element 141. That is, the functions provided by each of the switch element 154 and the adjustment element 141 can be realized by one element.

In some examplary embodiments, the introducing device 1 may also have an infusion port 1113 (see Figure 2) in communication with the liquid storage cavity 152. In this case, the user can inject the skin lotion into the liquid storage cavity 152 through the infusion port 1113. In some examplary embodiments, the infusion port 1113 may be disposed on an upper surface of the outer periphery 111c of the functional portion 111. For example, as shown in Figure 2, the upper surface of the outer periphery 111c of the functional portion 111 may be provided with a groove 1112 extending in the horizontal direction (the direction corresponding to the X-axis). A bottom surface of the groove 1112 is provided with the infusion port 1113 communicating with the liquid storage cavity 152. In some examplary embodiments, the infusion port 1113 may be shaped and sized to match a syringe used to inject the skin lotion. In this case, the user can inject the skin lotion from the infusion port 1113 into the liquid storage cavity 152 via a syringe.

In some examplary embodiments, the introducing device 1 may also have a cover 1114 for shielding the infusion port 1113. In some examplary embodiments, the cover 1114 may be shaped to match the groove 1112. In some examplary embodiments, the side, facing the infusion port 1113, of the cover 1114 may have a protrusion 11141 that cooperates with the infusion port 1113. In this case, the cover 1114 can serve to shield the infusion port 1113 more effectively. In some examplary embodiments, one end of the cover 1114 may be movably connected with the functional portion 111 (i.e., a movable connection end). The other end of the cover 1114 can be fixed in a clamping manner (i.e. a clamping structure end, wherein the clamping structure end can be the end opposite to the movably connected end). In this case, when the skin lotion is injected into the liquid storage cavity 152, the user can open the clamping structure to separate the clamping structure end of the cover 1114 from the functional portion 111, and rotate the cover 1114 along the movable connection end to open the cover 1114 to expose the infusion port 1113, whereby the user can inject the skin lotion into the liquid storage cavity 152 from the infusion port 1113.

In some examplary embodiments, the introducing device 1 requires an energy source to supply power during use. For this purpose, the introducing device 1 may also have a power module 16 for power supply (see Figure 4).

In some examplary embodiments, the power module 16 may be a battery or the like. In some examplary embodiments, the power module 16 may be arranged inside the introducing device 1. For example, the power module 16 may be disposed within the main body shell 10 (not shown). Alternatively, the power module 16 may be disposed within the base 20 (see Figure 7). In some examplary embodiments, the power module 16 may be a rechargeable powering device. For example, the power module 16 may be a rechargeable lithium battery. In this case, the introducing device 1 may also have a charging port 162 (see Figure 6) for connecting an external power supply to charge the power module 16. The power module 16 can thus be charged, enabling the introducing device 1 to be used continuously.

In other embodiments, the power module 16 may also be a power interface connected to an external power source to enable the external power source to supply power to the introducing device 1. Thereby, an external power supply can directly supply power to the introducing device 1.

In some examplary embodiments, as described above, the power module 16 may be connected to the control module 14, which may also be used to detect the amount of electricity information for power module 16. For example, if the power module 16 is a power supply device, the control module 14 may detect the remaining amount of electricity of the power supply device and generate electricity amount information.

In some examplary embodiments, the control module 14 may adjust the power module 16 according to the amount of electricity information. For example, in some examplary embodiments, if the power module 16 is a rechargeable power supply device and the power module 16 is in a state of charge, when the control module 14 detects that the acquired electricity amount information exceeds a predetermined threshold, the control module 14 may control the charging circuit of the power module 16 to disconnect to stop charging the power module 16. In this case, the use of the introducing device 1 can be made safer. In some examplary embodiments, the preset threshold may be determined on the basis of the maximum voltage at which the power module 16 is operating normally.

In some examplary embodiments, as described above, the control module 14 may also be used to inspect the working state of the power module 16. For example, in some examplary embodiments, if the power module 16 is a rechargeable power supply device, the control module 14 may inspect whether power module 16 is in a charging state or not, etc.

In some examplary embodiments, the control module 14 may adjust the output state of the stimulation source 13 on the basis of the acquired working state of the power module 16. In some examplary embodiments, if the power module 16 is a rechargeable powering device and the power module 16 is in a charging state, the control module 14 may send a control signal to the stimulation source 13 to cause the stimulation source 13 to stop outputting. That is, the stimulation source 13 does not provide electrical stimulation to the stimulation head 113 when the power module 16 is in the charging state. In this case, the use of the introducing device 1 can be made safer.

In some examplary embodiments, the control module 14 may also be connected to the atomizing device 153. The control module 14 can adjust the working state of the spraying device 15 according to the acquired information such as the working state of the power module 16. For example, if the power module 16 is in the charging state, the control module 14 may send a spraying control signal to the spraying device 15 to cause the spraying device 15 to discontinue spraying.

In some examplary embodiments, the introducing device 1 may also have a second display module 161 connected to the control module 14. In some examplary embodiments, the second display module 161 may be used to obtain the amount of electricity information and display for reflecting the remaining electricity amount of the power module 16. In some examplary embodiments, the second display module 161 may contain a plurality of second display lights, reflecting the remaining electricity amount of the power module 16 in different states of the second display lights. For example, in some examplary embodiments, the second display module 161 may comprise a plurality of second display lights, and the amount of electricity remaining in the power module 16 may be reflected by the number of lighted lights in the plurality of display lights. In other embodiments, the second display module 161 may comprise a second display light, which may reflect the remaining electricity amount of the power module 16 by a different color or flicker frequency of the second display light, etc. However, the embodiments of the present disclosure are not so limited, and the second display module 161 may also be other display devices. For example, the second display module 161 may include a display panel on which data directly reflects the remaining electricity amount of the power module 16, etc.

In some examplary embodiments, the second display module 161 may be disposed on an outer wall of the introducing device 1. For example, the second display module 161 may be disposed on an outer wall (not shown) of the main body shell 10. Alternatively, the second display module 161 may be disposed on the end face 20a of the base 20 (see Figure 6).

Figure 6 is a schematic diagram illustrating a structure of the base 20 according to an embodiment of the present disclosure at different viewing angles. Figure 7 is a block diagram illustrating a structure of the base 20 according to an embodiment of the present disclosure. Figure 8 is a schematic diagram illustrating a structure of the introducing device 1 according to an embodiment of the present disclosure. Herein, in the embodiments shown in Figures 6 and 8, detailed description can be made on the basis of an XYZ orthogonal coordinate system (see Figures 6(a) and 8(b)). The direction X may correspond to a horizontal left-right direction, the direction Y may correspond to a horizontal front-rear direction, and the direction Z may correspond to a vertical up-down direction. Figure 8(a) is a schematic diagram of the handheld device 10' separated from the base 20. Figure 8 (b) is a schematic diagram of the handheld device 10' secured to the base 20.

In embodiments to which the present disclosure relates, the shape of the base 20 may not be so limited, wherein the base 20 may be shaped for placement of the handheld device 10'. The related embodiments are to be describled in detail hereinafter with the reference to the accompanying drawings.

In some examplary embodiments, the introducing device 1 may also have a base 20 (see Figures 6 and 8) for matching with the main body shell 10. In this case, the introducing device 1 may include a handheld device 10' housed with the main body shell 10, and a base 20 for placing the handheld device 10'. Wherein the user may hold the handheld device 10' in hand to spray skin lotion or electrically stimulate the skin. In this case, the weight required to be held by the user in the introducing device 1 can be effectively reduced, and the operation by the user can be facilitated.

In some examplary embodiments, the base 20 may be substantially collumnar. For example, the base 20 may be substantially frustoconical. In some examplary embodiments, the end surface 20a close to the handheld device 10' may be no larger than the end surface 20b remote from the handheld device 10'. In some examplary embodiments, the transition between the end surface 20a and the end surface 20b may be smooth to form the base 20.

In some examplary embodiments, the end face 20a may be at a certain angle with respect to the horizontal direction. In some examplary embodiments, the height of the rear side of the end face 20a may be greater than the height of the front side thereof (see Figures 6 and 8). In this case, the handheld device 10' can be placed on the base 20 more stably.

In some examplary embodiments, the end face 20a may have a groove 21. In some examplary embodiments, the shape of the groove 21 may match the shape of the holding part 12. For example, the cross-sectional shape of the groove 21 in the horizontal direction may be circular.

In some examplary embodiments, the holding part 12 may be partially disposed within the groove 21 when the handheld device 10' is placed on the base 20. For example, the end, remote from the head part 11, of the holding part 12 may be placed within the groove 21 (see Figure 8). In some examplary embodiments, an inner diameter of the groove 21 may be sized to match an outer diameter of the holding part 12 disposed in the groove 21. In this case, when the holding part 12 is partially placed within the groove 21, the bottom and side walls of the groove 21 can provide support to the handheld device 10' such that the handheld device 10' is disposed substantially vertically on the base 20 (see Figure 8(b)).

In some examplary embodiments, the base 20 may also have a detection module 22 for detecting whether the handheld device 10' is placed within the groove 21 or not. Thereby, it is possible to detect whether the handheld device 10' is placed in the groove 21 or not. In some examplary embodiments, the detection module 22 may be an infrared pair of tubes disposed on the base 20. The infrared pair of tubes may include a transmitting tube and a receiving tube. For example, the transmitting tube may be an infrared transmitting tube and the receiving tube may be an infrared receiving tube or a photosensitive receiving tube.

In some examplary embodiments, the side walls of the groove 21 may be provided with two opposing windows 211 matching the infrared pair of tubes. In this case, if the handheld device 10' is placed in the groove 21, the infrared rays transmitted by the transmitting tube are blocked by the side wall of the handheld device 10', and the receiving tube cannot receive the infrared rays transmitted by the transmitting tube. If the handheld device 10' is removed from the groove 21, the infrared rays transmitted by the transmitting tube may be received by the receiving tube. Thus, it can be judged whether or not the handheld device 10' is placed in the groove 21 by whether or not the receiving tube receives infrared rays.

In some examplary embodiments, the base 20 may also have fixing device 202 for immobilization. For example, in some examplary embodiments, the fixing device 23 may be a suction cup disposed on the end surface 20b. In this case, the base 20 can be firmly fixed to, for example, a table top, and the base 20 can be prevented from being detached from the table top during use to avoid damage. In other embodiments, the fixing device 23 may also be other devices capable of securing the base 20. For example, the fixing device 23 may be a clip (not shown) disposed on the end face 20b or the side wall 20c of the base 20.

In some examplary embodiments, the base 20 may also have an auxiliary element 24 for adjusting the fixing device 23. In some examplary embodiments, the fixing action of the fixing device 23 can be adjusted by means of the auxiliary element 24. For example, in the embodiment shown in Figure 8, the fixing device 23 may be a suction cup disposed on the end face 20b, and the auxiliary element 24 may be a suction cup auxiliary element disposed on the side wall 20c of the base 20 adjacent to the end face 20b. By means of the suction cup auxiliary element it is possible to adjust whether the suction cup generates suction on, for example, a table top or the like, thereby adjusting whether the base 20 is fixed or not. In this case, it is convenient for the user to change the fixing position of the base 20 at any time, which is advantageous for the user to use it more conveniently.

In some examplary embodiments, as described above, the base 20 may have a first display module 131 and a second display module 161 (see Figure 6). Thereby, the user can know the amount of electricity and the output state of the introduucing device 1. In some examplary embodiments, the second display module 161 and the first display module 131 may be at different positions of the end face 20a respectively.

In some examplary embodiments, the power module 16 and/or the control module 14 and the like may be disposed within the base 20. In this case, the size and weight of the handheld device 10' can be reduced to facilitate use by the user.

In some examplary embodiments, if the power module 16 is disposed on the base 20, the handheld device 10' may be electrically connected to the base 20 via a transmission wire 30 (described later) (see Figures 6-8). For example, the stimulation source 13, the control module 14, and the spraying device 15, etc. may all be connected to the power module 16 (see Figure 9) such that the power module 16 can supply power to the above modules.. This enables the power module 16 to supply power to the handheld device 10'.

In some examplary embodiments, the base 20 may be connected to the handheld device 10' via the transmission wire 30, as described above. For example, one side of the transmission wire 30 may be connected to the end, remote from the head part 11, of the holding part 12, i.e., the lower end face 12b. The other side of the transmission wire 30 may be connected to the power module 16 disposed within the base 20.

In some examplary embodiments, the bottom surface of the groove 21 may be provided with a window 212 that matches the transmission wire 30. Thus, the transmission wire 30 can connect the handheld device 10' and the power module 16 provided within the base 20, respectively, through the window 212.

In some examplary embodiments, the transmission wire 30 may be a spring wire capable of conducting electricity. The transmission wire 30 can be automatically contracted without an external force. In other embodiments, the transmission wire 30 may be a flexible wire. The base 20 may have a winding mechanism 25 therein (see Figure 7) on which the transmission wire 30 may be wound. The winding mechanism 25 can control the transmission wire 30 to be wound back and wound on the winding mechanism 25 without external force. In this case, when the handheld device 10' is placed on the base 20, the transmission wire 30 can be automatically retracted into the base 20 through the window 212 (see Figure 6), and partially remains connected to the handheld device 10' through the window 212. When the handheld device 10' is removed from the base 20, the transmission wire 30 may be pulled out through the window 212 to maintain the handheld device 10' to be connected to the base 20. This enables the handheld device 10' to be placed on the base 20 more stably and neatly and, in use, ensures that the handheld device 10' is connected to the power module 16 to enable the power module 16 to supply power to the handheld device 10'.

Hereinafter, a method of using the introducing device 1 according to an example of the present embodiment will be described in detail with reference to the accompanying drawings. Figure 9 is a block diagram illustrating a structure of the introducing device 1 according to another embodiment of the present disclosure.

In some examplary embodiments, as described above, the introducing device 1 may have an introducing mode and a spraying mode.

In some examplary embodiments, the user may hold the introducing device 1 in hand and may treat the skin by adjusting the introducing device 1.

In some examplary embodiments, when the introducing device 1 is in the spraying mode, the user may hold the introducing device 1 with the spraying port 151 close to the skin to be treated, and skin lotion is sprayed to the surface of the skin by the spraying device 15 in the introducing device 1.

Specifically, when the introducing device 1 is in the spraying mode, the user can hold the introducing device 1 with the spraying port 151 close to the skin to be treated, and the spray is controlled by the switch element 154 so that the skin lotion is sprayed from the liquid storage cavity 152 through the spraying port 151 to the surface of the skin.

In some examplary embodiments, the spraying device 15 may also include an atomizing device 153 that may enable the skin lotion to be sprayed onto the surface of the skin in atomized form. Thereby, the skin lotion can be absorbed by the skin more effectively.

In some examplary embodiments, when the introducing device 1 is in the introducing mode, the user may hold the introducing device 1 in a hand so that at least one of the plurality of stimulation heads 113 contacts the surface of the skin, and the control module 14 may be enabled by the adjustment element 141 to control the stimulation source 13 to provide different intensities of electrical stimulation for stimulating the skin to the stimulation heads 13. In this case, hydrophilic channels are produced in the skin by electrical stimulation, whereby the skin lotion attached to the surface of the skin can permeate the skin via the hydrophilic channels. For example, at first, the user may turn on the stimulation source 13 through long pressing the adjustment element 141. Then, the user can enable the control module 14 to control and adjust the output state of the stimulation source 13 by short pressing the adjustment element 141. Finally, after the use of the introducing mode is completed, the user can turn off the stimulation source 13 by long pressing the adjustment element 141.

In some examplary embodiments, during using the introducing device 1 to electrically stimulate the skin, the user may keep the skin-contacting stimulation head 113 in a continuously moving state within a certain skin area. For example, the user may hold the introducing device 1 in hand to keep the stimulation head 113 in contact with the skin in a skin area to keep the stimulation head 113 in a continuous helical movement within the skin area. In this case, the use of the introducing device 1 can be made safer.

In some examplary embodiments, when the user uses the introducing device 1, the introducing device 1 may be firstly configured to spray the skin lotion onto the surface of the skin in the spraying mode, and then the introducing device 1 may be configured to electrically stimulate the skin to which the skin lotion is applied in the introducing mode.

In other examples, when a user uses the introducing device 1, the introducing device 1 may be configured to firstly electrically stimulate the skin in the introducing mode, and then the introducing device 1 may be configured to spray the skin lotion onto the surface of the electrically stimulated skin in the spraying mode.

In other examples, the user may also configure the introducing device 1 in both the spraying mode and the introducing mode when using the introducing device 1, i.e., the user may use the introducing device 1 to spray the skin lotion onto the skin while electrically stimulating the skin.

In some examplary embodiments, the introducing device 1 may include a handheld device 10' and a base 20. In some examplary embodiments, the user may fix the base 20 prior to using the introducing device 1, for example by controlling the fixing device 23 via the auxiliary element 24 to fix the base 20 to the table top. In some examplary embodiments, the user may hold the handheld device 10' in hand to spray the skin lotion onto the surface of the skin and to electrically stimulate the skin.

The invention is defined in the appended claims. Aspects, embodiments and examples disclosed herein which do not fall within the scope of the appended claims do not form part of the invention, and are merely provided for illustrative purposes. Furthermore, the methods presented in the present description are merely provided for illustrative purpose only and do not form part of the present invention.

## Claims

1. A handheld skin lotion introducing device (1), having
a stimulation head (113) which is in contact with surface of skin and electrically stimulates the skin so as to enable the skin to generate a hydrophilic channel,
a stimulation source (13) for providing electric stimulation to the stimulation head (113),
a control module (14) for controlling an output state of the stimulation source (13),
a spraying device (15) for spraying the skin lotion to the surface of the skin, the spraying device (15) is provided with a spraying port (151), a switch element (154) for controlling spraying ,a liquid storage cavity (152) communicated with the spraying port (151) and used for accomodating the skin lotion, and an atomizing device (153) for atomizing the skin lotion which is partially disposed within the introducing device (1),
an infusion port (1113) in communication with the liquid storage cavity (152) used for injecting the skin lotion into the liquid storage cavity (152), and
a cover (1114) having a protrusion (11141) cooperating with the infusion port (1113) which is serving to shield the infusion port (1113),
the introducing device (1) is provided with a spraying mode and an introducing mode, the introducing device (1) is configured such that, in the spraying mode, the spraying device (15) controls spraying with the switch element (154) to spray the skin lotion to the surface of the skin via the spraying port (151), and, in the introducing mode, the control module (14) controls the stimulation source (13) to provide the stimulation head (113) with an electric stimulation for stimulating the skin such that the skin lotion attached to the surface of the skin penetrates the skin via the hydrophilic channel,
wherein,
the atomizing device (153) includes an air pump (1531), an atomizing nozzle (1532) disposed at the spraying port (151), a mixing chamber (1533) disposed below the liquid storage cavity (152) and an obstruction portion 1534 for shielding the spraying port (151),
the air pump (1531) and the mixing chamber (1533) are provided with gas passages, and the obstruction portion (1534) movable thimble disposed in the mixing chamber (1533),
in the spraying mode,
the air pump (1531) generates pressurized gas which enters the mixing chamber (1533) from the gas passage to pressurize the skin lotion in the mixing chamber (1533) to be ejected from the spraying port (151),
the obstruction portion (1534) opens the spraying port (151) so that the skin lotion be sprayed through the spraying port (151) to the surface of the skin.

2. The introducing device according to claim 1, further including a power module (16) for powering.

3. The introducing device according to claim 2, wherein the control module (14) is also used for detecting the power module (16) to obtain working state and amount of electricity information of the power module (16).

4. The introducing device according to claim 3, wherein the power module (16) is rechargeable energy supply equipment, and if the control module (14) detects that the power module (16) is in a charging state, the control module (14) controls the stimulation source (13) to stop providing electric stimulation.

5. The introducing device according to claim 3, wherein the power module (16) is rechargeable energy supply equipment, and the introducing device (1) is provided with a charging port (162) connected with an external power supply and used for charging the power module (16), and if the power module (16) is in a charging state and the control module (14) obtains that the amount of electricity information of the power module (16) exceeds a preset threshold value, the control module (14) controls a charging circuit of the power module (16) to be disconnected.

6. The introducing device according to claim 1, further compr sing a main body shell (10), wherein the main body shell (10) comprises a head part (11) and a holding part (12) which is used for holding and is connected with the head part (11), and the stimulation head (113) and the spraying port (151) are arranged on the head part (11), the head part (11) includes a hollow, substantially collumnar functional portion (111) and a connecting portion (112) formed at an outer periphery of the functional portion (111), the holding part (12) and the connecting portion (112) being mechanically detachably connected to each other.

7. The introducing device according to claim 6, wherein the introducing device (1) further comprises a handheld device (10') formed by taking the main body shell (10) as a housing and a base (20) for placing the handheld device (10').

8. The introducing device according to claim 7, wherein the base (20) is further provided with a first display module (131) connected with the control module (14) and used for displaying the output state of the stimulation source (13) and a second display module (161) connected with the control module (14) and used for displaying the amount of electricity information.

9. The introducing device according to claim 7, wherein the base (20) comprises a groove (21) matching with the handheld device (10') and used for placing the handheld device (10').

10. The introducing device according to claim 9, wherein the base (20) is provided with a detection module (22) for detecting whether the handheld device (10') is placed in the groove (21) or not.

11. The introducing device according to claim 7, wherein the base (20) is provided with a power module (16) for supplying power, and the handheld device (10') is connected with the base (20) through a transmission wire (30).

12. The introducing device according to claim 11, wherein the base (20) is provided with a winding mechanism (25) used for matching the transmission wire (30), and the transmission wire (30) is configured to be wound on the winding mechanism (25).

13. The introducing device according to claim 7, wherein the base (20) is further provided with a fixing device (23) for immobilization.

## Patentansprüche

1. Handgehaltene Einführvorrichtung für Hautlotion (1), die aufweist
einen Stimulationskopf (113), der in Kontakt mit der Hautoberfläche steht und die Haut elektrisch stimuliert, um die Haut zu aktivieren, einen hydrophilen Kanal zu erzeugen,
eine Stimulationsquelle (13) zum Bereitstellen elektrischer Stimulation für den Stimulationskopf (113),
ein Steuermodul (14) zum Steuern eines Ausgabezustands der Stimulationsquelle (13),
eine Sprühvorrichtung (15) zum Sprühen der Hautlotion auf die Hautoberfläche, wobei die Sprühvorrichtung (15) mit einer Sprühöffnung (151), einem Schaltelement (154) zum Steuern des Sprühens, einem Flüssigkeitsspeicherraum (152), der mit der Sprühöffnung (151) in Verbindung steht und zum Aufnehmen der Hautlotion verwendet wird, und einer Zerstäubungsvorrichtung (153) zum Zerstäuben der Hautlotion, die teilweise innerhalb der Einführungsvorrichtung (1) angeordnet ist, versehen ist,
eine Infusionsöffnung (1113), die mit dem Flüssigkeitsspeicherraum (152) in Verbindung steht und zum Einspritzen der Hautlotion in den Flüssigkeitsspeicherraum (152) verwendet wird, und
eine Abdeckung (1114), die einen mit der Infusionsöffnung (1113) zusammenwirkenden Vorsprung (11141) aufweist, der dazu dient, die Infusionsöffnung (1113) abzuschirmen,
die Einführungsvorrichtung (1) mit einem Sprühmodus und einem Einführungsmodus versehen ist, die Einführungsvorrichtung (1) so konfiguriert ist, dass im Sprühmodus die Sprühvorrichtung (15) das Sprühen mit dem Schaltelement (154) steuert, um die Hautlotion über die Sprühöffnung (151) auf die Hautoberfläche zu sprühen, und im Einführungsmodus das Steuermodul (14) die Stimulationsquelle (13) steuert, um dem Stimulationskopf (113) eine elektrische Stimulation zur Stimulierung der Haut bereitzustellen, sodass die an der Hautoberfläche angebrachte Hautlotion über den hydrophilen Kanal in die Haut eindringt,
wobei,
die Zerstäubungsvorrichtung (153) eine Luftpumpe (1531), eine an der Sprühöffnung (151) angeordnete Zerstäubungsdüse (1532), eine unterhalb des Flüssigkeitsspeicherhohlraums (152) angeordnete Mischkammer (1533) und einen Obstruktionsabschnitt (1534) zur Abschirmung der Sprühöffnung (151) beinhaltet,
die Luftpumpe (1531) und die Mischkammer (1533) mit Gasdurchlässen versehen sind, und der Obstruktionsabschnitt (1534) bewegliche Fingerhut in der Mischkammer (1533) angeordnet ist,
im Sprühmodus,
die Luftpumpe (1531) Druckgas erzeugt, das durch den Gasdurchgang in die Mischkammer (1533) eintritt, um die Hautlotion in der Mischkammer (1533) unter Druck zu setzen, damit sie aus der Sprühöffnung (151) ausgestoßen wird,
der Obstruktionsabschnitt (1534) die Sprühöffnung (151) öffnet, sodass die Hautlotion durch die Sprühöffnung (151) auf die Hautoberfläche gesprüht wird.

2. Einführungsvorrichtung nach Anspruch 1, ferner beinhaltend ein Stromversorgungsmodul (16) zur Stromversorgung.

3. Einführungsvorrichtung nach Anspruch 2, wobei
das Steuermodul (14) ebenso dazu dient, das Stromversorgungsmodul (16) zu erfassen, um Informationen über den Arbeitszustand und die Strommenge des Stromversorgungsmoduls (16) zu erhalten.

4. Einführungsvorrichtung nach Anspruch 3, wobei
das Stromversorgungsmodul (16) eine wiederaufladbare Energieversorgungseinrichtung ist, und wenn das Steuermodul (14) erfasst, dass sich das Stromversorgungsmodul (16) in einem Ladezustand befindet, steuert das Steuermodul (14) die Stimulationsquelle (13) so, dass sie keine elektrische Stimulation mehr bereitstellt.

5. Einführungsvorrichtung nach Anspruch 3, wobei
das Stromversorgungsmodul (16) eine wiederaufladbare Energieversorgungseinrichtung ist, und die Einführungsvorrichtung (1) mit einem Ladeanschluss (162) versehen ist, der mit einer externen Stromversorgung verbunden ist und zum Laden des Stromversorgungsmoduls (16) verwendet wird, und wenn sich das Stromversorgungsmodul (16) in einem Ladezustand befindet und das Steuermodul (14) feststellt, dass die Menge an Elektrizitätsinformationen des Stromversorgungsmoduls (16) einen voreingestellten Schwellenwert überschreitet, steuert das Steuermodul (14), dass eine Ladeschaltung des Stromversorgungsmoduls (16) abgeschaltet wird.

6. Einführungsvorrichtung nach Anspruch 1, ferner umfassend eine Hauptkörperschale (10), wobei die Hauptkörperschale (10) umfasst
ein Kopfteil (11) und ein Halteteil (12), das zum Halten verwendet wird und mit dem Kopfteil (11) verbunden ist, und der Stimulationskopf (113) und die Sprühöffnung (151) an dem Kopfteil (11) angeordnet sind, das Kopfteil (11) einen hohlen, im Wesentlichen säulenförmigen Funktionsabschnitt (111) und einen an einem Außenumfang des Funktionsabschnitts (111) ausgebildeten Verbindungsabschnitt (112) beinhaltet, wobei das Halteteil (12) und der Verbindungsabschnitt (112) mechanisch lösbar miteinander verbunden sind.

7. Einführungsvorrichtung nach Anspruch 6, wobei
die Einführungsvorrichtung (1) ferner eine handgehaltene Vorrichtung (10') umfasst, die dadurch gebildet wird, dass die Hauptkörperschale (10) als Gehäuse und eine Basis (20) zum Aufstellen der handgehaltenen Vorrichtung (10') verwendet wird.

8. Einführungsvorrichtung nach Anspruch 7, wobei
die Basis (20) ferner mit einem ersten Anzeigemodul (131), das mit dem Steuermodul (14) verbunden ist und zur Anzeige des Zustands der Ausgabe der Stimulationsquelle (13) verwendet wird, und einem zweiten Anzeigemodul (161), das mit dem Steuermodul (14) verbunden ist und zur Anzeige der Strommenge verwendet wird, versehen ist.

9. Einführungsvorrichtung nach Anspruch 7, wobei
die Basis (20) eine Mulde (21) umfasst, die zu der handgehaltenen Vorrichtung (10') passt und zum Ablegen der handgehaltenen Vorrichtung (10') dient.

10. Einführungsvorrichtung nach Anspruch 9, wobei
die Basis (20) mit einem Erfassungsmodul (22) versehen ist, um zu erfassen, ob die handgehaltene Vorrichtung (10') in der Mulde (21) platziert ist oder nicht.

11. Einführungsvorrichtung nach Anspruch 7, wobei
die Basis (20) mit einem Stromversorgungsmodul (16) zur Stromversorgung versehen ist und die handgehaltene Vorrichtung (10') über einen Übertragungsdraht (30) mit der Basis (20) verbunden ist.

12. Einführungsvorrichtung nach Anspruch 11, wobei
die Basis (20) mit einem Wicklungsmechanismus (25) versehen ist, der zum Anpassen des Übertragungsdrahtes (30) verwendet wird, und der Übertragungsdraht (30) so konfiguriert ist, dass er auf den Wicklungsmechanismus (25) gewickelt wird.

13. Einführungsvorrichtung nach Anspruch 7, wobei
die Basis (20) ferner mit einer Befestigungsvorrichtung (23) zur Immobilisierung versehen ist.

## Revendications

1. Dispositif portatif d'introduction de lotion pour la peau (1), ayant
une tête de stimulation (113) qui est en contact avec la surface de la peau et stimule électriquement la peau de manière à permettre à la peau de générer un canal hydrophile,
une source de stimulation (13) pour fournir une stimulation électrique à la tête de stimulation (113),
un module de commande (14) pour commander un état de sortie de la source de stimulation (13),
un dispositif de pulvérisation (15) pour pulvériser la lotion cutanée à la surface de la peau, le dispositif de pulvérisation (15) est pourvu d'un orifice de pulvérisation (151), un élément de commutation (154) pour commander la pulvérisation, une cavité de stockage de liquide (152) communiquant avec l'orifice de pulvérisation (151) et utilisée pour loger la lotion pour la peau, et un dispositif d'atomisation (153) pour atomiser la lotion pour la peau qui est partiellement disposée au sein du dispositif d'introduction (1),
un orifice de perfusion (1113) en communication avec la cavité de stockage de liquide (152) utilisée pour injecter la lotion cutanée dans la cavité de stockage de liquide (152), et
un couvercle (1114) ayant une saillie (11141) coopérant avec l'orifice de perfusion (1113) qui sert à protéger l'orifice de perfusion (1113),
le dispositif d'introduction (1) est pourvu d'un mode de pulvérisation et d'un mode d'introduction, le dispositif d'introduction (1) est configuré de telle sorte que, en mode pulvérisation, le dispositif de pulvérisation (15) commande la pulvérisation avec l'élément de commutation (154) pour pulvériser la lotion pour la peau sur la surface de la peau par le biais de l'orifice de pulvérisation (151), et, en mode introduction, le module de commande (14) commande la source de stimulation (13) pour fournir à la tête de stimulation (113) une stimulation électrique pour stimuler la peau de sorte que la lotion cutanée fixée à la surface de la peau pénètre dans la peau par le biais du canal hydrophile,
dans lequel,
le dispositif d'atomisation (153) inclut une pompe à air (1531), une buse d'atomisation (1532) disposée au niveau de l'orifice de pulvérisation (151), une chambre de mélange (1533) disposée sous la cavité de stockage de liquide (152) et une partie d'obstruction 1534 pour protéger l'orifice de pulvérisation (151),
la pompe à air (1531) et la chambre de mélange (1533) sont pourvues de passages de gaz, et la virole mobile de la partie d'obstruction (1534) disposée dans la chambre de mélange (1533),
en mode pulvérisation,
la pompe à air (1531) génère un gaz sous pression qui entre dans la chambre de mélange (1533) depuis le passage de gaz pour pressuriser la lotion pour la peau dans la chambre de mélange (1533) pour être éjectée depuis l'orifice de pulvérisation (151),
la partie d'obstruction (1534) ouvre l'orifice de pulvérisation (151) de sorte que la lotion pour la peau soit pulvérisée à travers l'orifice de pulvérisation (151) jusqu'à la surface de la peau.

2. Dispositif d'introduction selon la revendication 1, incluant en outre un module d'alimentation (16) pour l'alimentation.

3. Dispositif d'introduction selon la revendication 2, dans lequel
le module de commande (14) est également utilisé pour détecter le module d'alimentation (16) pour obtenir l'état de fonctionnement et la quantité d'informations électriques du module d'alimentation (16).

4. Dispositif d'introduction selon la revendication 3, dans lequel
le module d'alimentation (16) est un équipement d'approvisionnement en énergie rechargeable, et si le module de commande (14) détecte que le module d'alimentation (16) est dans un état de charge, le module de commande (14) commande la source de stimulation (13) pour arrêter de fournir une stimulation électrique.

5. Dispositif d'introduction selon la revendication 3, dans lequel
le module d'alimentation (16) est un équipement d'approvisionnement en énergie rechargeable, et le dispositif d'introduction (1) est pourvu d'un port de charge (162) connecté à une alimentation électrique externe et utilisé pour charger le module d'alimentation (16), et si le module d'alimentation (16) est dans un état de charge et le module de commande (14) obtient que la quantité d'informations électriques du module d'alimentation (16) dépasse une valeur de seuil prédéfinie, le module de commande (14) commande un circuit de charge du module d'alimentation (16) pour qu'il soit déconnecté.

6. Dispositif d'introduction selon la revendication 1, comprenant en outre une coque de corps principal (10), dans lequel la coque de corps principal (10) comprend
une pièce de tête (11) et une pièce de maintien (12) qui est utilisée pour maintenir et est connectée à la pièce de tête (11), et la tête de stimulation (113) et l'orifice de pulvérisation (151) sont agencés sur la pièce de tête (11), la pièce de tête (11) inclut une partie creuse fonctionnelle sensiblement columnaire (111) et une pièce de connexion (112) formée à une périphérie externe de la partie fonctionnelle (111), la pièce de maintien (12) et la partie de connexion (112) étant connectées mécaniquement de manière amovible l'une à l'autre.

7. Dispositif d'introduction selon la revendication 6, dans lequel
le dispositif d'introduction (1) comprend en outre un dispositif portatif (10') formé en prenant la coque de corps principal (10) comme un logement et une base (20) pour placer le dispositif portatif (10').

8. Dispositif d'introduction selon la revendication 7, dans lequel
la base (20) est en outre pourvue d'un premier module d'affichage (131) connecté au module de commande (14) et utilisé pour afficher l'état de sortie de la source de stimulation (13) et d'un deuxième module d'affichage (161) connecté au module de commande (14) et utilisé pour afficher la quantité d'informations électriques.

9. Dispositif d'introduction selon la revendication 7, dans lequel
la base (20) comprend une rainure (21) correspondant au dispositif portatif (10') et utilisée pour placer l'appareil portatif (10').

10. Dispositif d'introduction selon la revendication 9, dans lequel
la base (20) est pourvue d'un module de détection (22) pour détecter si l'appareil portatif (10') est placé dans la rainure (21) ou non.

11. Dispositif d'introduction selon la revendication 7, dans lequel
la base (20) est pourvue d'un module d'alimentation (16) pour fournir une alimentation, et le dispositif portatif (10') est connecté à la base (20) par le biais d'un fil de transmission (30).

12. Dispositif d'introduction selon la revendication 11, dans lequel
la base (20) est pourvue d'un mécanisme d'enroulement (25) utilisé pour faire correspondre le fil de transmission (30), et le fil de transmission (30) est configuré pour être enroulé sur le mécanisme d'enroulement (25).

13. Dispositif d'introduction selon la revendication 7, dans lequel
la base (20) est en outre pourvue d'un dispositif de fixation (23) pour l'immobilisation.
